# EUROPEAN PATENT SPECIFICATION

(11) **EP 3 123 988 B1**
(45) Date of publication and mention of the grant of the patent: **23.05.2018**
(21) Application number: 15178915.3
(22) Date of filing: 29.07.2015
(51) Int. Cl.: A61F 13/00

(54) **BIOLOGICAL FIBER COMPOSITE DRESSING**
VERBAND AUS BIOLOGISCHEN FASERVERBUNDSTOFF
PANSEMENT COMPOSITE EN FIBRE BIOLOGIQUE

(43) Date of publication of application: 01.02.2017
(73) Proprietor: Harvest Belle Biotech, New Taipai City 248 (TW)
(72) Inventor: LU, Chi-Hsiang, 248 New Taipei City (TW)
(74) Representative: Epping - Hermann - Fischer

(56) References cited:
- JP-A- 2009 007 721
- US-A- 4 742 164
- US-A- 5 144 021
- US-A1- 2009 209 897
- US-A1- 2013 004 784

## Description

### BACKGROUND OF THE INVENTION

### 1. Field of the Invention

The present invention relates to biological fiber composite dressings, and more particularly, to a biological fiber composite dressing applied to the skin.

### 2. Description of the Related Art

In the current medical field, a cotton pad or gauze is mostly used as the dressing for the care of a wound. However, such type of dressing has some drawbacks, for example, a poor antibacterial property, a high possibility of a wound infection, easiness for developing wound adhesion, and difficulty to be removed.

Afterwards, the cotton pads and gauzes have been replaced with non-woven dressings, since the non-woven dressings have the characteristics of better absorbency and being capable of providing a moist environment to aid of the wound repair. However, when the liquid or moisture absorbed by the non-woven dressings is gradually reduced, the issue of wound adhesion is likely to arise.

On the other hand, in addition to the basic living requirements, the modern people pay more attention to cosmetic skin care, especially for the facial care. Hence, the beauty industry focuses on the demands of the facial care, and develops a variety of facial mask products. There are a variety of masks, such as a mud paste-type mask, a tear-peel type mask, a sheet-like mask, and so on.

Although the mud paste-type mask contains some ingredients or minerals for skin care, the mask has to be washed off after application. Hence, the ingredients for skin care are hard to be really absorbed by the skin. Further, because the mud paste-type mask contains more minerals, more preservatives must be added to prevent bacteria from growing in the moist mud paste. The tear-peel type mask has main ingredients, such as, polymer gel, water and alcohol, and promotes the blood circulation of the skin by increasing the epidermal temperature. However, since the tear-peel type mask is no peeled off until being dry, it might cause damage to the sensitive skin during the peeling of the mask. In addition, the tear-peel-type mask does not contain any moisturizing ingredients for the dryness of the mask, such that it is not appropriate for the dry skin. The sheet-like mask is a monolayer sheet absorbed with essence with specific functions, and it can be used for a variety of skin cares by adjusting the ingredients. Although the sheet-like mask does not need to be washed off after application, the mask does not have any cleaning effect. The above sheet-like mask is mostly made of a monolayer of non-woven fabric. For a user to apply the non-woven fabric soaked with the essence, a higher concentration of essence is required due to the rapid water evaporation in the non-woven fabric. Consequently, the essence is wasted, while the problem of water evaporation remains unresolved.

Therefore, the beauty industry has developed a biological fiber membrane as a mask. Although the biological fiber membrane has a better moisturizing property, the overall attaching comfort is poor. Besides, the entire sheet of the biological fiber membrane is not easy to expand after absorption with the essence. Thus, there is still a need to develop a novel dressing product.

US 2013/004784 A1 relates to multi-phase biomaterials based on bacterially synthesized nanocellulose and a method for producing same.

JP 2009/007721 relates to a biological fiber formed inside cloth.

US 4,742,164 discloses a bacterial cellulose-containing molding material having high dynamic strength.

US 5,144,021 relates to a conventionally reticulated cellulose.

US 2009/0209897 teaches a photoactivated antimicrobial wound dressing.

### SUMMARY OF THE INVENTION

In view of the above disadvantages of the prior art, the present invention provides a biological fiber composite dressing according to ciaim1. Preferred embodiments are set forth in the subclaims.

The biological fiber composite dressing of the present invention has a cloth membrane and a biological fiber membrane, so as to enable the dressing to absorb more moisture or active ingredients by the biological fiber membrane. Since the biological fiber membrane has more delicate pores than the cloth membrane, it can prevent moisture from rapidly evaporating from the cloth membrane while having gas permeability, and thereby enhancing the moisturizing property of the dressing. Therefore, when the biological fiber composite dressing of the present invention is applied to the skin, for example, when it is used as a mask, the dressing does not need to absorb excess active ingredients. As a result, cost is saved substantially for the end-users.

### BRIEF DESCRIPTION OF THE DRAWINGS

FIG. 1 is a schematic diagram of the structure of a biological fiber composite dressing of the present invention;
FIGs. 2A and 2B show scanning electron microscope (SEM) photographs of the biological fiber composite dressing of the present invention, wherein FIG. 2A shows a photograph of the three-dimensional reticular structure extended to the cloth membrane, and FIG. 2B shows a side view of the biological fiber membrane;
FIGs. 3A and 3B show an SEM photograph taken at 500X magnification of the biological fiber membrane side of the biological fiber composite dressing of the present invention and an SEM photograph at 500 X magnification of a conventional biological fiber membrane, respectively;
FIG. 4 shows a schematic diagram of the biological fiber composite dressing of the present invention having an additional biological fiber membrane; and
FIG. 5 shows results of a permeability test on the biological fiber composite dressing of the present invention and the conventional biological fiber membrane, wherein FIG. 5(a) shows the test result of the biological fiber composite dressing of the present invention, and FIG. 5(b) shows the test result of the conventional biological fiber membrane.

### DETAILED DESCRIPTION OF THE PREFERRED EMBODIMENTS

The following specific examples are used for illustrating the present invention. One skilled in the art can easily conceive the other advantages and effects of the present invention, from the disclosure of the present specification.

It should be noted that all of the drawings depict a structure, proportion, size, etc., are only used to match the specification for a person skilled in the art to understand and reads. It is not intended to limit the conditions which can be implemented in the present invention, such that it is not substantially meaningful technically. Any modification of the structure, change in the proportion or adjustment of the size are be within the scope encompassed in the technical contents disclosed in the present invention. At the same time, terms, such as "first," "second," "on" and "a/an" etc., are merely to facilitate the understanding of the descriptions, and should not be construed to limit the implemental scope of the present invention. Any change or adjustment of the relationships is also considered to be within the implemental scope, without any substantial changes to the technical content.

The term "parallel" used herein means the morphology of which a plurality of backbone fibers are in the same direction, such as a longitudinal direction or a width direction.

The present invention provides a biological fiber composite dressing, including a cloth membrane having a first surface and an opposing second surface, wherein the cloth membrane has cloth membrane fibers; and a biological fiber membrane having a third surface and an opposing fourth surface, wherein the biological fiber membrane is formed by bacteria of the genus *Gluconacetobacter,* and the fourth surface layer is combined with the first surface layer, and the fourth surface layer has a plurality of biological fibers being wound and combined with the cloth membrane fibers extending therealong.

The cloth membrane is a woven fabric or non-woven fabric. In general, the woven fabric is woven by textile technologies. The woven fabric is woven by using artificial fibers or natural fibers, or blending with the artificial fibers and the natural fibers. In a specific example, the artificial fibers are polyester, acrylics or nylon. The natural fibers are silk or cotton fibers.

The biological fiber membrane of the present invention is obtained by culturing microorganisms. It is found in the present invention that the biological fiber membrane formed by culturing bacteria of the genus *Gluconacetobacter* in a medium containing mannitol, peptone, yeast extract and agar has a plurality of biological fibers being wound and combined with the cloth membrane fibers.

In an embodiment for producing the biological fiber membrane, the fermentation of a strains takes place. First of all, a culture medium is provided in a container, and the culture medium contains some known components selected from gelatin, gum arabic, agar, and etc. The culture medium still needs some carbon sources, such as mannitol, glucose, and etc., and other components, such as peptone and yeast extract. The weight ratio of the carbon source, peptone and yeast extract is in a range from 5:1:1 to 4:1:1. Subsequently, the pH value of the culture medium is preferably controlled at acidic, such as between pH 0.5 to 6. The initial concentration of microorganisms can be controlled in a range of 102 to 105 bacteria/ml. The stationary culturing of the microorganisms is performed at 25 to 28°C for 24 to 96 hours. Later, the aforesaid cloth membrane is placed flat in the culture medium. After 24 to 48 hours, a composite dressing is taken out, and the biological fiber composite dressing of the present invention is obtained.

After testing, the biological fiber membrane in the biological fiber composite dressing of the present invention has a plurality of biological fibers, and the amount of the biological fibers per unit area is from 0.005 g/cm² to 0.008 g/cm². The diameter of each of the biological fibers is from 20 nm to 100 nm.

In addition, after drying the biological fiber composite dressing of the present invention and then re-absorbing water and active ingredients, the biological fiber composite dressing has at least 50% of rehydration rate, at least 60% of rehydration rate, at least 70% of rehydration rate, even up to 91% of rehydration rate. In another aspect, the thickness of the biological fiber membrane of the biological fiber composite dressing is at least 20 µm, such as from 20 µm to 30 µm, or from 20 µm to 26 µm, or from 24 µm to 26 µm.

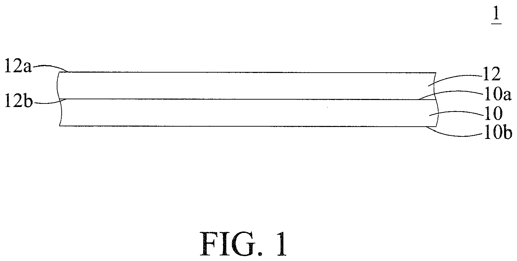

As shown in FIG. 1, a biological fiber composite dressing 1 of the present invention includes a cloth membrane 10 having a first surface layer 10a and an opposing second surface layer 10b; and a biological fiber membrane 12 disposed on the first surface layer 10a. The biological fiber membrane 12 has a third surface layer 12a and a fourth surface layer 12b, and the fourth surface layer 12b is combined with the first surface 10a.

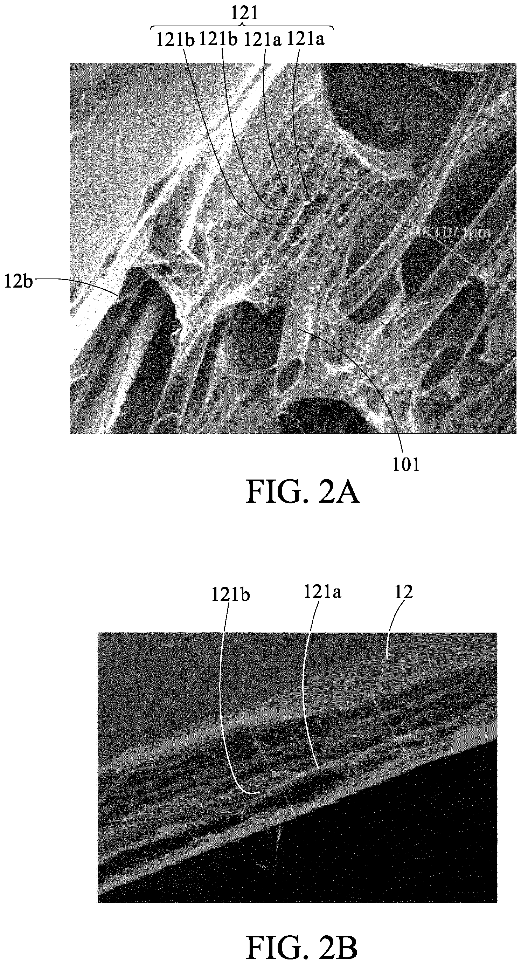

In addition, as shown in FIG. 2A, a plurality of biological fibers being wound and combined with the cloth membrane fibers 101 are extended along the fourth surface layer 12b of the biological fiber membrane 12.

As shown in FIG. 2A, the biological fiber membrane 12 has a three-dimensional reticular structure 121, and is extended to the interior of the first surface layer 10a of the cloth membrane 10 from the fourth surface layer 12b. The three-dimensional reticular structure 121 is composed of a plurality of biological fibers. More particularly, the three-dimensional reticular structure 121 has a plurality of backbone fibers 121a parallel to each other and a plurality of inter-layer fibers 121b interwoven at any two of the adjacent backbone fibers 121a. Therefore, any two of the adjacent backbone fibers 121a are linked in the horizontal and vertical directions, to form the three-dimensional reticular structure 121. Both of the backbone fibers 121a and the inter-layer fibers 121b are biological fibers. As shown in FIG. 2A, the diameter of each of the backbone fibers 121a is greater than or equal to the diameter of each of the inter-layer fibers 121b. Even, it can be found that the diameter of each of the backbone fibers 121a is smaller than the diameter of each of the cloth membrane fibers 101.

As shown in FIG. 2B, a side view of the biological fiber membrane 12 is provided, wherein the biological fiber membrane 12 also has a plurality of backbone fibers 121a, which are parallel to each other or extended along a longitudinal direction or a width direction of the biological fiber membrane 12, and a plurality of inter-layer fibers 121b interwoven with the backbone fibers 121a. Besides, the three-dimensional reticular structure is bound between two surface layers of the biological fiber membrane 12. The density of the three-dimensional reticular structure is smaller than the density of the two surface layers, for example, the density of the third surface and the fourth surface layer. In this example, the thickness of the biological fiber membrane of the biological fiber composite dressing is from 20 µm to 30 µm.

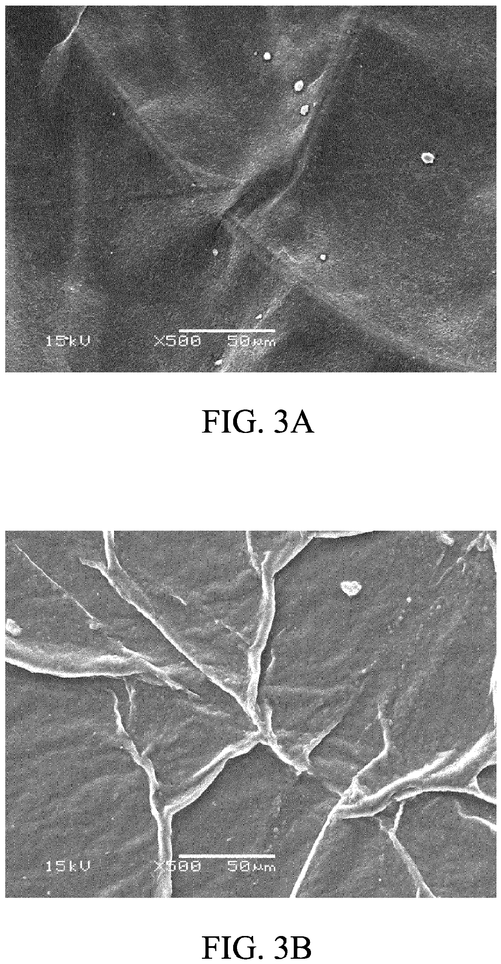

As shown in FIG. 3A and 3B, an SEM photograph at 500 X magnification of the biological fiber membrane side of the biological fiber composite dressing of the present invention and a SEM photograph at 500 X magnification of the conventional biological fiber membrane, respectively, are provided.

As shown in FIG. 3A, the biological fiber membrane side of the biological fiber composite dressing of the present invention is flat. The strips shown in FIG. 3A are the cloth membrane fibers underneath the biological fiber membrane. Hence, the biological fiber membrane side of the biological fiber composite dressing of the present invention is very flat. On the contrary, the surface of the conventional biological fiber membrane shown in FIG. 3B has many foldings. Therefore, poor attachment of such biological fiber membrane to the skin affects touch, and also results in poor absorbency of drugs or active ingredients.

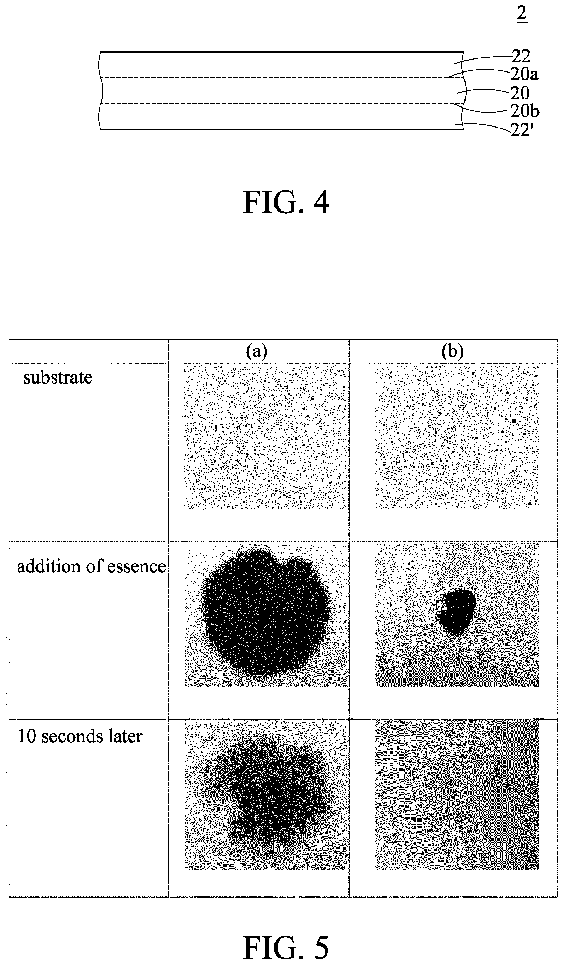

As shown in FIG. 4, if the cloth membrane 20 is provided in the container for a longer period during culturing or the cloth membrane 20 is flipped over, an additional biological fiber membrane 22' is formed on the second surface layer 20b of the cloth membrane 20. In this example of the biological fiber composite dressing 2 of the present invention, in addition to the biological fiber membrane 22 formed on the first surface layer 20a, an additional biological fiber membrane 22' is further included.

The biological fiber composite dressing of the present invention is provided to apply to the skin. It is particularly useful as a mask. Thus, the biological fiber composite dressing of the present invention can further includes an active ingredient or a drug. The examples of the active ingredient include humectants, whitening ingredients, anti-wrinkle ingredients, exfoliating ingredients, growth factors and enzymes. Additions of different active ingredients enable the biological fiber composite dressing of the present invention to have the effects for stress relief and body massage.

### A tensile strength test on the biological fiber composite dressing of the present invention

The biological fiber composite dressing of the present invention was cut into test pieces each with a size of 15mm x 15mm, and then three test pieces were subjected to a tensile strength test. The obtained results were 2113.70 g/15mm, 2145.00 g/15mm and 1951.90 g/15mm, respectively. The average value of the tensile strength is 2070.20 g/15mm.

The conventional biological fiber membrane was subjected to the same test, and its tensile strength was up to 2607.33 g/15mm. It can be seen that the biological fiber composite dressing of the present invention has better pliability than the conventional biological fiber membrane.

### An elongation test on the biological fiber composite dressing of the present invention

In addition, the biological fiber composite dressing of the present invention was subjected to an elongation test, and the obtained results were 21.00%, 16.20% and 16.60%, respectively. The average value of the elongation was 17.93%. The conventional biological fiber membrane was subjected to the same test, but its elongation was only 5.66%. It is thus clear that the elongation for the biological fiber composite dressing of the present invention is significantly better, and the biological fiber composite dressing can be extended and attached to a larger area.

### A water absorbency test on the biological fiber composite dressing of the present invention

Three test pieces, each with a dimension of 100mm x 15mm, were subjected to a water absorbency test. First, deionized water was injected in a dish, and kept the water temperature at 23±°C. Then, the test pieces were contacted with water to measure a water absorption height for one minute.

The results show that the average height of the water absorption for the biological fiber composite dressing of the present invention was 40 mm, while the average height for the conventional biological fiber membrane was 1 mm. Thus, the biological fiber composite dressing of the present invention has better water absorbency.

### Measurements for the amount of the biological fibers per unit area in the biological fiber composite dressing of the present invention

The biological fiber composite dressing of the present invention was cut into 16 pieces, each with a size of 5 cm x 5 cm. The pieces were dried at 60°C for 10 minutes, and then weighed for their dry weights. The dry weight of each of the pieces after deducting the dry weight of the cloth membrane was divided by area of the piece. The obtained amounts of the biological fibers per unit area were from 0.005 g/cm² to 0.008 g/cm².

In comparison, the amount of the biological fibers per unit area of the conventional biological fiber membrane was only 0.001 g/cm².

### A rehydration rate test of the biological fiber composite dressing of the present invention

The wet weights of the biological fiber composite dressing of the present invention and the conventional biological fiber membrane were measured. Then, the dressing and the biological fiber membrane were dried at 20°C for 30 minutes. After the dressing and the biological fiber membrane were curly, white and filamentous, the dry weights were measured. Subsequently, the dressing and the biological fiber membrane were contacted with water for 30 minutes, and the weights after rehydration were recorded. The rehydration rate was obtained by dividing the weight after rehydration by the weight difference between the wet weight and the dry weight.

It was found that the biological fiber composite dressing of the present invention had a rehydration rate of at least 50%, and at most 91%. By contrast, the conventional biological fiber membrane could not absorb water. The reason was that the amount of the biological fibers per unit area in the conventional biological fiber membrane was only 0.001 g/cm², while the amount of the biological fibers per unit area in the biological fiber membrane of the biological fiber composite dressing of the present invention was from 0.005 g/cm² to 0.008 g/cm². Thus, the biological fiber composite dressing of the present invention has an excellent rehydration rate.

### Permeability test on the biological fiber composite dressing of the present invention

The biological fiber composite dressing of the present invention and the conventional biological fiber membrane were placed flat, on a substrate, and then the same amount of stained essences was added onto the biological fiber composite dressing of the present invention and the conventional biological fiber membrane, respectively. After 10 seconds, the permeability of the substrate was observed visually.

As shown in FIG. 5, FIG. 5(a) shows the test result of the biological fiber composite dressing of the present invention, and FIG. 5(b) shows the test result of the conventional biological fiber membrane. Among these results, when the essences were just added onto the conventional biological fiber membrane, the essences had poor spreading. After 10 seconds, the essences could not penetrate effectively thorough the substrate. By contrast, the biological fiber composite dressing of the present invention had better diffusibility and significant permeability.

In summary, the biological fiber composite dressing of the present invention includes a cloth membrane and a biological fiber membrane, so as to enable the biological fiber membrane to absorb more moisture or active ingredients. Since the biological fiber membrane has more delicate pores than the cloth membrane, it can prevent moisture from rapidly evaporating from the cloth membrane while having gas permeability, and thereby enhancing the moisturizing property of the dressing. In another aspect, when one side of the biological fiber composite dressing of the present invention is the cloth membrane, the dressing has an attaching comfort and a long-lasting moisturizing property at the same time. Therefore, when the biological fiber composite dressing of the present invention is applied to the skin, for example, when it is used as a mask, the dressing does not need to absorb excess active ingredients. As a result, the cost is saved substantially for the end-users.

While the examples are used to illustrate the principle of the present invention and the effect being brought about, they are not intended to limit the preset invention. Any one skilled in the art can make modifications to the examples above without substantially departing from the scope of the present invention. Therefore, the scope of the present invention should be accorded to the appended claims.

## Claims

1. A biological fiber composite dressing (1, 2), comprising:
a cloth membrane (10, 20) having a first surface layer (10a, 20a) and an opposing second surface layer (10b, 20b), wherein the cloth membrane comprises cloth membrane fibers (101); and
a biological fiber membrane (12, 22) formed by bacteria of the genus *Gluconacetobacter,* wherein the biological fiber membrane (12, 22) has a third surface layer (12a) and an opposing fourth surface layer (12b), and wherein the fourth surface layer (12b) is combined with the first surface layer (10a, 20a), and the fourth surface layer (12b) comprises a plurality of biological fibers being wound and combined with the cloth membrane fibers (101) extending therealong,
wherein the biological fiber membrane (12, 22) further comprises a three-dimensional reticular structure (121) bound between the third surface layer (12a) and the fourth surface layer (12b), and wherein the density of the three-dimensional reticular structure (121) is smaller than the density of the third surface layer (12a) and the fourth surface layer (12b), and the three-dimensional reticular structure (121) extends to the interior of the first surface layer (10a, 20a) of the cloth membrane (10, 20) from the fourth surface layer (12b), and the three-dimensional reticular structure (121) has a plurality of backbone fibers (121a) parallel to each other and a plurality of inter-layer fibers (121b) interwoven at any two of adjacent backbone fibers.

2. The biological fiber composite dressing (1, 2) of claim 1, wherein the three-dimensional reticular structure (121) is composed of the plurality of biological fibers.

3. The biological fiber composite dressing (1, 2) of claim 1, wherein a diameter of each of the plurality of backbone fibers (121a) is greater than or equal to a diameter of each of the plurality of inter-layer fibers (121b).

4. The biological fiber composite dressing (1, 2) of claim 1, wherein the plurality of backbone fibers (121a) extend along a longitudinal direction or a width direction of the biological fiber membrane (12, 22).

5. The biological fiber composite dressing (1, 2) of claim 1, wherein the cloth membrane (10, 20) is a woven fabric, and wherein the woven fabric is woven by using an artificial fiber or a natural fiber, or blended with the artificial fiber and the natural fiber.

6. The biological fiber composite dressing (1, 2) of claim 5, wherein the artificial fiber is selected from the group consisting of polyester, acrylics and nylon.

7. The biological fiber composite dressing (1, 2) of claim 5, wherein the natural fiber is one of silk and a cotton fiber.

8. The biological fiber composite dressing (1, 2) of claim 1, wherein the cloth membrane (10, 20) is a non-woven fabric.

9. The biological fiber composite dressing (1, 2) of claim 1, further comprising an additional biological fiber membrane (22') disposed on the second surface (20b).

10. The biological fiber composite dressing (1, 2) of claim 9, wherein the additional biological fiber membrane (22') has a plurality of biological fibers being wound and combined with the cloth membrane fibers.

11. The biological fiber composite dressing (1, 2) of claim 1, further comprising an active ingredient or a drug.

12. The biological fiber composite dressing (1, 2) of claim 11, wherein the active ingredient is selected from the group consisting of a humectant, a whitening ingredient, an anti-wrinkle ingredient, an exfoliating ingredient, a growth factor, and an enzyme.

13. The biological fiber composite dressing (1, 2) of claim 1, wherein the biological fiber membrane (12, 22) is formed by culturing the bacteria of the genus *Gluconacetobacter* in a culture medium having mannitol, peptone, yeast extract, and agar.

14. The biological fiber composite dressing (1, 2) of claim 1, wherein the amount of the biological fibers of the biological fiber membrane (12, 22) per unit area is from 0.005 g/cm² to 0.008 g/cm².

15. The biological fiber composite dressing (1, 2) of claim 14, wherein the diameter of each of the biological fiber is from 20 nm to 100 nm.

16. The biological fiber composite dressing (1, 2) of claim 1, wherein the biological fiber composite dressing (1, 2) has a rehydration rate of at least 50% determined with the method in the description by dividing a weight after rehydration by the weight difference between a wet weight and a dry weight of the biological fiber composite dressing (1, 2).

17. The biological fiber composite dressing (1, 2) of claim 16, wherein the biological fiber composite dressing (1, 2) has a rehydration rate of at least 50% to 91%.

18. The biological fiber composite dressing (1, 2) of claim 1, wherein a thickness of the biological fiber membrane (12, 22) is at least 20 µm.

19. The biological fiber composite dressing (1, 2) of claim 18, wherein the thickness of the biological fiber membrane (12, 22) is from 20 µm to 30 µm.

## Patentansprüche

1. Biologischer Faserverbundstoff-Verband (1, 2), umfassend:
eine Stoffmembran (10, 20) mit einer ersten Oberflächenschicht (10a, 20a) und einer entgegengesetzten zweiten Oberflächenschicht (10b, 20b), wobei die Stoffmembran Stoffmembranfasern (101) aufweist; und
eine biologische Fasermembran (12, 22), gebildet durch Bakterien der Gattung *Gluconacetobacter,* wobei die biologische Fasermembran (12, 22) eine dritte Oberflächenschicht (12a) und eine entgegengesetzte vierte Oberflächenschicht (12b) hat, und wobei die vierte Oberflächenschicht (12b) mit der ersten Oberflächenschicht (10a, 20a) kombiniert ist, und die vierte Oberflächenschicht (12b) eine Vielzahl von biologischen Fasern aufweist, die mit den sich daran erstreckenden Stoffmembranfasern (101) verwickelt und kombiniert sind,
wobei die biologische Fasermembran (12, 22) ferner eine zwischen der dritten Oberflächenschicht (12a) und der vierten Oberflächenschicht (12b) eingebundene dreidimensionale netzartige Struktur (121) umfasst, und wobei die Dichte der dreidimensionalen netzartigen Struktur (121) kleiner als die Dichte der dritten Oberflächenschicht (12a) und der vierten Oberflächenschicht (12b) ist, und sich die dreidimensionale netzartige Struktur (121) von der vierten Oberflächenschicht (12b) ins Innere der ersten Oberflächenschicht (10a, 20a) der Stoffmembran (10, 20) erstreckt, und die dreidimensionale netzartige Struktur (121) eine Vielzahl von zueinander parallelen Gerüstfasern (121a) und eine Vielzahl von an jeweils zwei der benachbarten Gerüstfasern verflochtenen Zwischenschichtfasern (121b) hat.

2. Biologischer Faserverbundstoff-Verband (1, 2) nach Anspruch 1, wobei die dreidimensionale netzartige Struktur (121) aus der Vielzahl von biologischen Fasern zusammengesetzt ist.

3. Biologischer Faserverbundstoff-Verband (1, 2) nach Anspruch 1, wobei ein Durchmesser von jeder der Vielzahl von Gerüstfasern (121a) größer als oder gleich einem Durchmesser von jeder der Vielzahl von Zwischenschichtfasern (121b) ist.

4. Biologischer Faserverbundstoff-Verband (1, 2) nach Anspruch 1, wobei sich die Vielzahl von Gerüstfasern (121a) entlang einer Längsrichtung oder einer Breitenrichtung der biologischen Fasermembran (12, 22) erstreckt.

5. Biologischer Faserverbundstoff-Verband (1, 2) nach Anspruch 1, wobei die Stoffmembran (10, 20) ein Gewebe ist, und wobei das Gewebe unter Verwendung einer Kunstfaser oder einer Naturfaser gewebt ist, oder mit der Kunstfaser und der Naturfaser vermischt ist.

6. Biologischer Faserverbundstoff-Verband (1, 2) nach Anspruch 5, wobei die Kunstfaser ausgewählt ist aus der Gruppe bestehend aus Polyester, Acryl und Nylon.

7. Biologischer Faserverbundstoff-Verband (1, 2) nach Anspruch 5, wobei die Naturfaser eine Seide oder eine Baumwollfaser ist.

8. Biologischer Faserverbundstoff-Verband (1, 2) nach Anspruch 1, wobei die Stoffmembran (10, 20) ein Vliesstoff ist.

9. Biologischer Faserverbundstoff-Verband (1, 2) nach Anspruch 1, ferner umfassend eine zusätzliche biologische Fasermembran (22'), die auf der zweiten Oberfläche (20b) angeordnet ist.

10. Biologischer Faserverbundstoff-Verband (1, 2) nach Anspruch 9, wobei die zusätzliche biologische Fasermembran (22') eine Vielzahl von biologischen Fasern hat, die mit den Stoffmembranfasern verwickelt und kombiniert sind.

11. Biologischer Faserverbundstoff-Verband (1, 2) nach Anspruch 1, ferner aufweisend einen Wirkstoff oder ein Medikament.

12. Biologischer Faserverbundstoff-Verband (1, 2) nach Anspruch 11, wobei der Wirkstoff ausgewählt ist aus der Gruppe bestehend aus einem Feuchthaltemittel, einem Aufhellungszusatz, einem Antifaltenzusatz, einem Exfoliationszusatz, einem Wachstumsfaktor und einem Enzym.

13. Biologischer Faserverbundstoff-Verband (1, 2) nach Anspruch 1, wobei die biologische Fasermembran (12, 22) durch Kultivierung der Bakterien der Gattung *Gluconacetobacter* in einem Kulturmedium mit Mannitol, Pepton, Hefeextrakt und Agar gebildet wird.

14. Biologischer Faserverbundstoff-Verband (1, 2) nach Anspruch 1, wobei die Menge der biologischen Fasern der biologischen Fasermembran (12, 22) pro Flächeneinheit von 0,005 g/cm² bis 0,008 g/cm² ist.

15. Biologischer Faserverbundstoff-Verband (1, 2) nach Anspruch 14, wobei der Durchmesser von jeder der biologischen Fasern von 20 nm bis 100 nm ist.

16. Biologischer Faserverbundstoff-Verband (1, 2) nach Anspruch 1, wobei der biologische Faserverbundstoff-Verband (1, 2) eine Rehydrationsrate von mindestens 50 % hat, bestimmt mit dem in der Beschreibung aufgeführten Verfahren durch Teilen eines Gewichts nach Rehydration durch die Gewichtsdifferenz zwischen einem Nassgewicht und einem Trockengewicht des biologischen Faserverbundstoff-Verbands(l, 2).

17. Biologischer Faserverbundstoff-Verband (1, 2) nach Anspruch 16, wobei der biologische Faserverbundstoff-Verband (1, 2) eine Rehydrationsrate von mindestens 50 % bis 91 % hat.

18. Biologischer Faserverbundstoff-Verband (1, 2) nach Anspruch 1, wobei eine Dicke der biologischen Fasermembran (12, 22) mindestens 20 µm ist.

19. Biologischer Faserverbundstoff-Verband (1, 2) nach Anspruch 18, wobei die Dicke der biologischen Fasermembran (12, 22) von 20 µm bis 30 µm ist.

## Revendications

1. Pansement composite en fibres biologiques (1, 2), comprenant :
une membrane en tissu (10, 20) présentant une première couche de surface (10a, 20a) et une deuxième couche de surface opposée (10b, 20b), dans lequel la membrane en tissu comprend des fibres de membrane en tissu (101), et
une membrane en fibres biologiques (12, 22) formée par des bactéries du genre *Gluconacetobacter,* dans lequel la membrane en fibres biologiques (12, 22) présente une troisième couche de surface (12a) et une quatrième couche de surface opposée (12b), et dans lequel la quatrième couche de surface (12b) est combinée avec la première couche de surface (10a, 20a), et la quatrième couche de surface (12b) comprend une pluralité de fibres biologiques enroulées et combinées avec les fibres de membrane en tissu (101) s'étendant le long d'elles,
dans lequel la membrane en fibres biologiques (12, 22) comprend en outre une structure réticulaire tridimensionnelle (121) liée entre la troisième couche de surface (12a) et la quatrième couche de surface (12b), et dans lequel la densité de la structure réticulaire tridimensionnelle (121) est inférieure à la densité de la troisième couche de surface (12a) et de la quatrième couche de surface (12b), et la structure réticulaire tridimensionnelle (121) s'étend jusqu'à l'intérieur de la première couche de surface (10a, 20a) de la membrane en tissu (10, 20) à partir de la quatrième couche de surface (12b), et la structure réticulaire tridimensionnelle (121) présente une pluralité de fibres d'ossature (121a) parallèles entre elles et une pluralité de fibres de couche intermédiaire (121b) entrelacées au niveau de deux fibres quelconques parmi des fibres d'ossature adjacentes.

2. Pansement composite en fibres biologiques (1, 2) selon la revendication 1, dans lequel la structure réticulaire tridimensionnelle (121) est composée de la pluralité de fibres biologiques.

3. Pansement composite en fibres biologiques (1, 2) selon la revendication 1, dans lequel un diamètre de chacune de la pluralité de fibres d'ossature (121a) est supérieur ou égal à un diamètre de chacune de la pluralité des fibres de couche intermédiaire (121b).

4. Pansement composite en fibres biologiques (1, 2) selon la revendication 1, dans lequel la pluralité de fibres d'ossature (121a) s'étend le long d'une direction longitudinale ou d'une direction de largeur de la membrane en fibres biologiques (12, 22).

5. Pansement composite en fibres biologiques (1, 2) selon la revendication 1, dans lequel la membrane en tissu (10, 20) est un tissu tissé, et dans lequel le tissu tissé est tissé en utilisant une fibre artificielle ou une fibre naturelle, ou mélangé avec la fibre artificielle et la fibre naturelle.

6. Pansement composite en fibres biologiques (1, 2) selon la revendication 5, dans lequel la fibre artificielle est sélectionnée parmi le groupe consistant en le polyester, les acryliques et le nylon.

7. Pansement composite en fibres biologiques (1, 2) selon la revendication 5, dans lequel la fibre naturelle est un élément parmi la soie et une fibre de coton.

8. Pansement composite en fibres biologiques (1, 2) selon la revendication 1, dans lequel la membrane en tissu (10, 20) est un tissu non tissé.

9. Pansement composite en fibres biologiques (1, 2) selon la revendication 1, comprenant en outre une membrane en fibres biologiques supplémentaire (22') disposée sur la seconde surface (20b).

10. Pansement composite en fibres biologiques (1, 2) selon la revendication 9, dans lequel la membrane en fibres biologiques supplémentaire (22') présente une pluralité de fibres biologiques enroulées et combinées avec les fibres de membrane en tissu.

11. Pansement composite en fibres biologiques (1, 2) selon la revendication 1, comprenant en outre un ingrédient actif ou un médicament.

12. Pansement composite en fibres biologiques (1, 2) selon la revendication 11, dans lequel l'ingrédient actif est sélectionné parmi le groupe consistant en un humectant, un agent de blanchiment, un ingrédient antirides, un ingrédient exfoliant, un facteur de croissance, et une enzyme.

13. Pansement composite en fibres biologiques (1, 2) selon la revendication 1, dans lequel la membrane en fibres biologiques (12, 22) est formée en cultivant les bactéries du genre *Gluconacetobacter* dans un milieu de culture présentant du mannitol, de la peptone, un extrait de levure, et de l'agar-agar.

14. Pansement composite en fibres biologiques (1, 2) selon la revendication 1, dans lequel la quantité des fibres biologiques de la membrane en fibres biologiques (12, 22) par unité de surface s'étend de 0,005 g/cm² à 0,008 g/cm².

15. Pansement composite en fibres biologiques (1, 2) selon la revendication 14, dans lequel le diamètre de chaque fibre biologique s'étend de 20 nm à 100 nm.

16. Pansement composite en fibres biologiques (1, 2) selon la revendication 1, dans lequel le pansement composite en fibres biologiques (1, 2) présente un taux de réhydratation d'au moins 50 % déterminé avec le procédé de la description en divisant un poids après réhydratation par la différence de poids entre un poids humide et un poids sec du pansement composite en fibres biologiques (1, 2).

17. Pansement composite en fibres biologiques (1, 2) selon la revendication 16, dans lequel le pansement composite en fibres biologiques (1, 2) présente un taux de réhydratation d'au moins 50 % à 91 %.

18. Pansement composite en fibres biologiques (1, 2) selon la revendication 1, dans lequel une épaisseur de la membrane en fibres biologiques (12, 22) est d'au moins 20 µm.

19. Pansement composite en fibres biologiques (1, 2) selon la revendication 18, dans lequel l'épaisseur de la membrane en fibres biologiques (12, 22) s'étend de 20 µm à 30 µm.
